# EUROPÄISCHE PATENTANMELDUNG

(11) **EP 1 247 535 A1**
(43) Veröffentlichungstag der Anmeldung: **09.10.2002**
(21) Anmeldenummer: 02007573.5
(22) Anmeldetag: 03.04.2002
(51) Int. Cl.: A61L 9/02, A61L 9/03, A61L 9/04, A61L 9/12, A61L 9/18

(54) **Elektrische Duftlampe**

(30) Priorität: 04.04.2001 DE 10116764
(71) Anmelder: hydrosun Medizintechnik GmbH, 79379 Müllheim (DE)
(72) Erfinder: Braun, Werner, 6062 Wilen (CH)
(74) Vertreter: Schnekenbühl, Robert Matthias L.

(57) **Zusammenfassung**

Die vorliegende Erfindung betrifft eine Vorrichtung zur Freisetzung eines Duftstoffes umfassend ein Gefäß zur Aufnahme eines flüssigen oder festen Grundstoffes sowie eine Strahlungsquelle, wobei die Strahlungsquelle so auf das Gefäß einwirken kann, dass ein in dem Grundstoff enthaltener Duftstoff austreten kann sowie ein Verfahren zur Freisetzung eines Duftstoffes aus einem Grundstoff.

Bei dem erfindungsgemäßen Verfahren ist vorgesehen, dass die Strahlungsquelle ein elektrisches Leuchtmittel ist.

## Beschreibung

Die vorliegende Erfindung betrifft eine Vorrichtung zur Freisetzung eines Duftstoffes umfassend ein Gefäß zur Aufnahme eines flüssigen oder festen Grundstoffes sowie eine Strahlungsquelle, wobei die Strahlungsquelle so auf das Gefäß einwirken kann, dass ein in dem Grundstoff enthaltener Duftstoff austreten kann sowie ein Verfahren zur Freisetzung eines Duftstoffes aus einem Grundstoff.

Es sind Vorrichtungen der zuvor genannten Gattung bekannt, bei denen die Strahlungsquelle eine Wachskerze oder ein Petroleumbrenner oder dergleichen ist. Die Verwendung derartiger Strahlungsquellen hat den Nachteil, dass diese in ihrer Leistung nur eingeschränkt regelbar sind und vergleichsweise unkomfortabel in Betrieb genommen werden müssen, da hierzu ein offenes Feuer notwendig ist.

Der Erfindung liegt das Problem zugrunde, eine Vorrichtung nach dem Oberbegriff des Patentanspruchs 1 bereitzustellen, die leichter zu regeln und komfortabler in Betrieb zu nehmen ist.

Dieses Problem wird durch eine Vorrichtung nach Patentanspruch 1 gelöst. Erfindungsgemäß ist vorgesehen, dass die Strahlungsquelle ein elektrisches Leuchtmittel ist. Eine Leistungsregelung kann hier durch einen handelsüblichen Dimmer erfolgen. Das Ein- und Ausschalten kann wie bei elektrischen Betriebsmitteln üblich über einen elektrischen Ein- und Ausschalter recht komfortabel erfolgen.

In einer Weiterbildung der Erfindung ist vorgesehen, dass das elektrische Leuchtmittel im Zusammenwirken mit einem Reflektor betrieben werden kann. Ein Reflektor bündelt die durch das elektrische Leuchtmittel abgegebene Strahlung in eine Richtung und erhöht so die Beleuchtungsstärke in dieser Richtung.

Vorzugsweise sind der Reflektor und das Leuchtmittel gemeinsam in einem Gehäuse angeordnet. Das Gehäuse ermöglicht sowohl eine Befestigung als auch einen Schutz von Reflektor und Leuchtmittel vor Beschädigung.

In einer weiteren Ausführungsform ist vorgesehen, dass zwischen dem Gefäß und dem elektrischen Leuchtmittel ein Filter angeordnet ist. Mit dem Filter können unerwünschte Strahlungsbestandteile heraus gefiltert werden.

Vorzugsweise ist vorgesehen, dass der Filter Wärmestrahlung heraus filtert. Die auf das Gefäß auftreffende Strahlung bewirkt in diesem Fall keine Wärmezufuhr.

Weiter ist vorgesehen, dass der Filter ein H₂O-Filter ist. Es ist dies ein handelsüblicher Filter zum Herausfiltern von Wärmestrahlungsbestandteilen.

Des weiteren kann vorgesehen sein, dass der Filter in seinen optischen Eigenschaften veränderbar ist. Abhängig von dem durch das Leuchtmittel ausgesandten Spektrum kann durch Veränderung des Filters das auf das Gefäß auftreffende Spektrum variiert werden.

Vorteilhaft sind die optischen Eigenschaften des Filters elektrisch veränderbar. Mit dem elektrischen Schaltmittel kann eine einfache und reproduzierbare Verstellung erreicht werden.

In einer weiteren Ausgestaltung ist vorgesehen, dass die veränderbaren optischen Eigenschaften des Filters insbesondere die Wellenlänge ist, die von dem Filter gesperrt wird. Auf diese Weise können einzelne Wellenlänge gezielt aus dem Spektrum, das auf das Gefäß auftrifft, herausgefiltert werden.

Das eingangs genannte Problem wird außerdem von einem Verfahren zur Freisetzung eines Duftstoffes aus einem Grundstoff nach Patentanspruch 10 gelöst. Erfindungsgemäß ist vorgesehen, dass der Grundstoff einer Strahlung ausgesetzt wird, die eine chemische Veränderung des Grundstoffes bewirkt und die chemische Veränderung den Duftstoff freisetzt. Durch Veränderung der Strahlungsintensität und des Strahlungsspektrums kann die Menge des freigesetzten Duftstoffes verändert werden.

In einer Ausgestaltung des Verfahrens ist vorgesehen, dass der Grundstoff abhängig von der auf diesen einwirkenden Strahlung unterschiedliche Duftstoffe freisetzt. Ohne Veränderung an dem Grundstoff vornehmen zu müssen können so unterschiedliche Duftstoffe freigesetzt werden. Ebenso ist es möglich, zeitabhängig verschiedene Duftstoffe freizusetzen.

Der Grundstoff ist vorzugsweise eine wässrige Lösung. Wässrige Lösungen sind handhabungssicher und gegenüber zum Beispiel alkoholischen Lösungen ungefährlich.

Im Folgenden wird ein Ausführungsbeispiel der Erfindung beschrieben. Dabei zeigt:

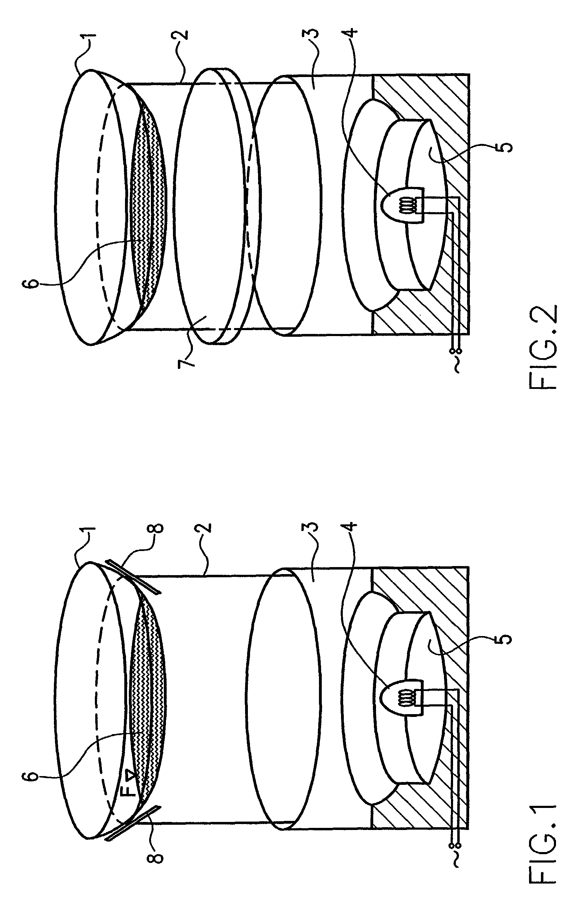

- Figur 1: eine Prinzipdarstellung einer erfindungsgemäßen Vorrichtung.

Eine Schale 1 gemäß Figur 1 wird getragen durch ein Gestell 2. Die Schale 1 kann z. B. eine Glasschale in Form einer Kugelkappe oder dergleichen sein. Hier sind grundsätzlich nahezu alle teilweise oder vollständig geöffneten Gefäße einsetzbar. Das Gestell 2 ist im dargestellten Ausführungsbeispiel ein Drahtgestell.

Das Gestell 2 ist verbunden mit einem Gehäuse 3, in dem ein elektrisches Leuchtmittel 4 sowie ein Reflektor 5 angeordnet sind. Das elektrische Leuchtmittel 4 sowie der Reflektor 5 sind so angeordnet, dass die von dem elektrischen Leuchtmittel 4 erzeugte Strahlung im wesentlichen auf die Unterseite der Schale 1 auftrifft.

In der Schale 1 befindet sich eine Flüssigkeit 6. Diese kann z.B. eine Lösung, Emulsion oder Mischung mehrerer Stoffe sein. Statt der Flüssigkeit 6 können hier alternativ feste Gegenstände, beispielsweise ein Granulat, eingefüllt sein. Durch die Bestrahlung der Unterseite der Schale 1 mittels des elektrischen Leuchtmittels 4 in Verbindung mit dem Reflektor 5 erwärmt sich die Schale 1 sowie die Flüssigkeit 6, so dass ein in der Flüssigkeit 6 enthaltener Duftstoff verdampft.

Alternativ oder in Ergänzung zur Ausnutzung der Wärmestrahlung des elektrischen Leuchtmittels 4 und dessen Absorption durch die Flüssigkeit 6 beziehungsweise die Schale 1 kann die Wellenlänge der von dem elektrischen Leuchtmittel 4 ausgestrahlten Strahlung so auf die Eigenschaften der Flüssigkeit 6 abgestimmt sein, dass die Einwirkung der von dem elektrischen Leuchtmittel 4 ausgehenden Strahlung unmittelbar eine chemische Veränderung der Flüssigkeit 6, beispielsweise eine Spaltung eines dort enthaltenden Stoffes, auslöst. Der chemisch veränderte Stoff wird aus der Flüssigkeit 6 freigesetzt, während der nicht veränderte Stoff nicht freigesetzt wird. Durch die Einwirkung der von dem elektrischen Leuchtmittel 4 ausgesandten Strahlung auf die Flüssigkeit 6 wird auf diese Weise eine Substanz freigesetzt und in dem Raum, in dem sich die gesamte Vorrichtung befindet, verteilt.

Figur 2 zeigt eine gegenüber der Darstellung der Figur 1 erweiterte Ausführung der Vorrichtung. Zwischen dem Gehäuse 3 und der Schale 1 ist zusätzlich ein Filter 7 angeordnet. Es kann sich hier beispielsweise um einen handelsüblichen H₂O-Filter handeln. Dieser filtert diejenigen Strahlungsbestandteile, die zu einer Erwärmung von Wasser führen, heraus. Die sich in der Schale 1 befindende Flüssigkeit 6 wird daher nicht erwärmt, so dass nur die Strahlungsbestandteile, die eine chemische Veränderung der darin enthaltenen Flüssigkeit bewirken, durchgelassen werden. Auf diese Weise kann z. B. eine zu starke Verdunstung von Flüssigkeitsanteilen mit einem Siedepunkt deutlich höher als Raumtemperatur vermieden werden.

Der Filter 7 kann zusätzlich bezüglich seiner optischen Eigenschaften veränderbar sein. Beispielsweise könnte der Filter 7 durch elektrische Schaltmittel, die hier nicht dargestellt sind, bezüglich seines Durchlassspektrums veränderbar sein. Unabhängig von der Intensität der Strahlung des elektrischen Leuchtmittels 4 kann so eine Regelung der aus der Schale 1 austretenden Gasmenge bewirkt werden. Enthält die Flüssigkeit 6 Bestandteile, die sich bei unterschiedlichen Strahlungswellenlängen chemisch verändern und dabei unterschiedliche Geruchsnuancen ausprägen, wird eine in der Art der Geruchsfreisetzung veränderbare Vorrichtung geschaffen.

Zusätzlich können an der Schale 1 Spiegel 8 angeordnet sein, die durch die Bestrahlung seitens des elektrischen Leuchtmittels 4 in Verbindung mit dem Reflektor 5 einen Lichteffekt in der Umgebung der Vorrichtung erzeugen. Alternativ könnten beispielsweise auch Teile der Schale 1 verspiegelt sein.

### BEZUGSZEICHENLISTE

- 1: Schale
- 2: Gestell
- 3: Gehäuse
- 4: Leuchtmittel
- 5: Reflektor
- 6: Flüssigkeit
- 7: Filter
- 8: Spiegel

## Patentansprüche

1. Vorrichtung zur Freisetzung eines Duftstoffes umfassend ein Gefäss (1) zur Aufnahme eines flüssigen oder festen Grundstoffes (6) sowie eine Strahlungsquelle, wobei die Strahlungsquelle so auf das Gefäss (1) einwirken kann, dass ein in dem Grundstoff (6) enthaltener Duftstoff (6) austreten kann
**dadurch gekennzeichnet, dass**
die Strahlungsquelle ein elektrisches Leuchtmittel (4) ist.

2. Vorrichtung nach Anspruch 1,
**dadurch gekennzeichnet, dass**
das elektrische Leuchtmittel (4) in Zusammenwirken mit einem Reflektor (5) betrieben werden kann.

3. Vorrichtung nach einem der Ansprüche 1 oder 2,
**dadurch gekennzeichnet, dass**
der Reflektor (5) und das Leuchtmittel (4) gemeinsam in einem Gehäuse (3) angeordnet sind.

4. Vorrichtung nach einem der Ansprüche 1 bis 3,
**dadurch gekennzeichnet, dass**
zwischen dem Gefäss (1) und dem elektrischen Leuchtmittel (4) ein Filter (7) angeordnet ist.

5. Vorrichtung nach einem der Ansprüche 1 bis 4,
**dadurch gekennzeichnet, dass**
der Filter (7) Wärmestrahlung herausfiltert.

6. Vorrichtung nach einem der Ansprüche 1 bis 5,
**dadurch gekennzeichnet, dass**
der Filter (7) ein H₂O-Filter ist.

7. Vorrichtung nach einem der Ansprüche 1 bis 6,
**dadurch gekennzeichnet, dass**
der Filter (7) in seinen optischen Eigenschaften veränderbar ist.

8. Vorrichtung nach Anspruch 7,
**dadurch gekennzeichnet, dass**
die optischen Eigenschaften des Filters (7) elektrisch veränderbar sind.

9. Vorrichtung nach einem der Ansprüche 7 oder 8,
**dadurch gekennzeichnet, dass**
die veränderbaren optischen Eigenschaften des Filters (7) insbesondere die Wellenlänge ist, die von dem Filter (7) gesperrt wird.

10. Verfahren zur Freisetzung eines Duftstoffes aus einem Grundstoff,
**dadurch gekennzeichnet, dass**
der Grundstoff einer Strahlung ausgesetzt wird, die eine chemische Veränderung des Grundstoffes bewirkt und die chemische Veränderung den Duftstoff freisetzt.

11. Verfahren nach Anspruch 10,
**dadurch gekennzeichnet, dass**
der Grundstoff abhängig von der auf diesen einwirkenden Strahlung unterschiedliche Duftstoffe freisetzt.

12. Verfahren nach einem der Ansprüche 10 oder 11,
**dadurch gekennzeichnet, dass**
der Grundstoff eine wässrige Lösung ist.
